# NOUVEAU FASCICULE DE BREVET EUROPEEN

(11) **EP 0 597 022 B2**
(45) Date de publication et mention de la décision concernant l'opposition: **17.11.1999**
(45) Mention de la délivrance du brevet: 03.04.1996
(21) Numéro de dépôt: 92917543.8
(22) Date de dépôt: 30.07.1992
(51) Int. Cl.: A61K 7/00, A61K 7/13

(54) **UTILISATION POUR LA TEINTURE TEMPORAIRE DES FIBRES KERATINIQUES D'UN PIGMENT INSOLUBLE OBTENU PAR POLYMERISATION OXYDANTE DE DERIVES INDOLIQUES**
VERWENDUNG VON EINEM DURCH EXIDATIVER POLYMERISATION VON INDOLDERIVATEN ERZEUGTEN UNLÖSLICHEN PIGMENT ZUR ZEITLICHEN FÄRBUNG VON KERATINISCHEN FASERN
PROCESS FOR THE TEMPORARY DYEING OF KERATIN FIBRES INVOLVING AN INSOLUBLE PIGMENT OBTAINED BY THE OXIDATIVE POLYMERISATION OF INDOLE DERIVATIVES

(30) Priorité: 01.08.1991 FR 9109823
(43) Date de publication de la demande: 18.05.1994
(73) Titulaire: L'OREAL, 75008 Paris (FR)
(72) Inventeur: ANDREAN, Hervé, F-75014 Paris (FR); JUNINO, Alex, F-93190 Livry-Gargan (FR); LEZORAY, Louis, F-93190 Livry-Gargan (FR); COTTERET, Jean, F-78480 Verneuil-sur-Seine (FR); AUDOUSSET, Marie Pascale, F-92300 Levallois-Perret (FR); MAUBRU, Mireille, F-78400 Chatou (FR)
(74) Mandataire: Casalonga, Axel
(86) Numéro de dépôt international: FR9200745
(87) Numéro de publication internationale: WO9302655

(56) Documents cités:
- EP-A- 0 313 380
- EP-A- 0 376 776
- EP-A- 0 379 409
- EP-A- 0 467 767
- WO-A-90/01919
- DE-A- 3 824 094
- GB-A- 2 207 153

## Description

La présente invention est relative à un procédé de teinture temporaire des fibres kératiniques mettant en oeuvre une composition contenant au moins un pigment insoluble obtenu par polymérisation oxydante de dérivés indoliques.

On connaît essentiellement trois types de procédés de coloration des fibres kératiniques tels que les cheveux qui sont constitués par :
a) la coloration dite permanente qui a pour fonction d'apporter une modification sensible de la couleur naturelle et qui met en oeuvre des colorants d'oxydation qui pénètrent dans la fibre kératinique et forment le colorant par un processus de condensation oxydative ;
b) la coloration semi permanente ou directe, cette coloration ne met pas en oeuvre le processus de condensation oxydative et résiste à 4 à 5 shampooings.
c) la coloration temporaire ou fugace donne lieu à une modification légère de la couleur naturelle de la chevelure qui tient d'un shampooing à l'autre et qui sert à embellir ou corriger une nuance déjà obtenue.

L'invention est relative à un procédé de coloration du dernier ty-type conduisant à une coloration éliminable au premier shampooing. On peut également l'assimiler à un procédé "de maquillage des fibres kératiniques".

Pour modifier et embellir de manière temporaire les cheveux, on a déjà proposé, une teinture avec des colorants directs, mais cette teinture peut être hétérogène sur les parties des cheveux qui ont été abîmées par les différentes dégradations dues aux traitements tels que par exemple les permanentes, la chaleur, ou aux agents atmosphériques (soleil, intempéries). Par ailleurs ces colorants ne permettent généralement pas d'obtenir une nuance grise naturelle et esthétique sauf en utilisant un mélange avec d'autres colorants, lesquels présentent cependant l'inconvénient d'avoir souvent des résistances à la lumière ou aux shampooings différentes, ce qui peut donner lieu à des colorations finales non satisfaisantes. Enfin ce type de colorants n'estompe pas suffisamment les cheveux blancs ; ce qui constitue un autre inconvénient.

On a également proposé d'utiliser des polymères colorés formés par greffage d'un ou plusieurs colorants de nature azoïque, triphénylméthanique, azinique, indoaminique ou anthraquinonique sur une chaîne polymère. Ces polymères colorés ne sont pas totalement satisfaisants notamment au niveau de l'homogénéité de la coloration obtenue, de sa résistance, sans compter les problèmes liés à la fabrication de ce type de polymères colorés, notamment en ce qui concerne leur reproductibilité.

On a proposé d'utiliser une poudre inorganique comportant des pigments mélaniques formés par polymérisation oxydante de dérivés indoliques (GB-A-2 207 153) pour le maquillage de la peau et la coloration des cheveux. Egalement, dans EP-A-0 379 409 on a utilisé produits à base de particules de polymère comportant des pigments mélaniques, formés in situ par oxydation d'un colorant indolique, son procédé de préparation et son utilisation en cosmétique. WO-A-90/01919 décrit une colorante por la peau formé par la polymerisation oxydante d'un hydroxyindole. EP-A-0 313 380 décrit compositions mélaniques formées in situ sur particules polymériques.
Aussi, EP-A-0 467 767 est relative à un produit sous forme de poudre constitué de particules à structure lamellaires, organiques ou minérales, ayant une dimension inférieure à 50 micrometers et comportant au moins un pigment mélanique synthétique, formé in situ par oxydation d'un composé indolique, et à son utilisation pour la protection de l'épiderme humain, dans les produits de maquillage et pour la coloration des cheveux.

L'objectif de la demanderesse a été de trouver un moyen de colorer de façon temporaire les fibres kératiniques, en particulier les cheveux. Elle a recherché notamment des compositions permettant de déjaunir les cheveux blancs pour leur donner une nuance grise naturelle et esthétique. La coloration doit en particulier estomper les cheveux blancs, l'agent colorant doit donc être suffisamment substantif pour masquer les cheveux blancs sans l'être de trop, en particulier sur les fibres sensibilisées, afin d'être facilement éliminable au premier shampooing tout en résistant à des brossages multiples et aux frottements et ne pas tâcher les vêtements et les oreillers.

On connaît par ailleurs les colorants indoliques et notamment le 5, 6-dihydroxyindole qui ont déjà été préconisés pour teindre les fibres kératiniques notamment les cheveux, en mettant en oeuvre un processus de développement par voie oxydative soit en présence de l'oxygène de l'air, soit en présence de différents agents ou catalyseurs d'oxydation. Il s'agit dans ce cas d'une coloration que l'on peut assimiler à la coloration dite permanente dans la mesure où le colorant se développe à l'intérieur de la fibre à la suite de l'action des agents ou catalyseurs d'oxydation.

La présente invention a pour objet l'application sur les fibres kératiniques d'un pigment insoluble résultant de la polymérisation oxydante d'un dérivé indolique.

On appelle pigment insoluble un pigment insoluble dans les milieux cosmétiquement acceptables utilisés pour la coloration temporaire. La demanderesse a découvert que les compositions contenant de tels pigments permettaient de conférer aux fibres kératiniques, et notamment aux cheveux, une coloration temporaire éliminable au premier shampooing mais résistant cependant à des brossages, coiffages multiples, aux frottements et ne tachant pas les vêtements. Les compositions conformes à l'invention contenant les pigments définis ci-après et se présentant notamment sous forme de gels, permettent d'obtenir une coloration qui ne poudre pas au brossage.
Le pigment utilisé conformément à l'invention présente vis-à-vis des fibres kératiniques, une substantivité suffisamment élevée pour masquer et estomper les cheveux blancs.

L'invention a pour objet l'utilisation d'un pigment insoluble issu de la polymérisation oxydante d'un composé indolique, pour la coloration temporaire des fibres kératiniques.

L'invention a également pour objet des compositions destinées à être utilisées dans un procédé de coloration temporaire des fibres kératiniques.

D'autres objets de l'invention apparaîtront à la lecture de la description et des exemples qui suivent.

Le pigment utilisé conformément à l'invention, pour la coloration temporaire des fibres kératiniques, est caractérisé par le fait qu'il s'agît d'un pigment insoluble résultant de la polymérisation oxydante d'un composé indolique répondant à la formule (I) : 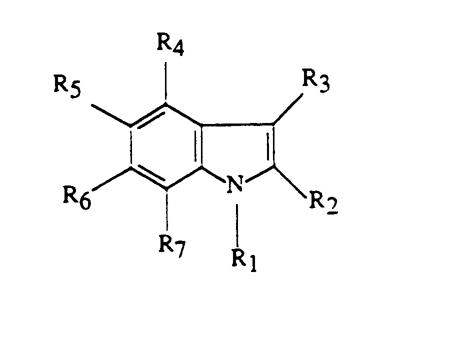 dans laquelle :
- R¹ et R³ représentent, indépendamment l'un de l'autre, un atome d'hydrogène ou un groupe alkyle en C₁-C₄;
- R² représente un atome d'hydrogène, un groupe alkyle en C₁-C₄, un groupe carboxyle ou un groupe alcoxy (C₁-C₄)-carbonyle ;
- R⁴ et R⁷ représentent, indépendamment l'un de l'autre, un atome d'hydrogène, un groupe hydroxy, un groupe alkyle en C₁-C₄, un groupe amino, un groupe alcoxy en C₁-C₄, un groupe acyl (C₂-C₄)-oxy ou un groupe acyl (C₂-C₄)-amino ;
- R⁵ représente un atome d'hydrogène, un groupe hydroxy, un groupe alcoxy en C₁-C₄, un groupe alkyle en C₁-C₄, un atome d'halogène, un groupe amino, un groupe acyl (C₂-C₁₄)-oxy, un groupe acyl (C₂-C₄)-amino ou un groupe triméthylsilyloxy ;
- R⁶ représente un atome d'hydrogène, un groupe hydroxy, un groupe alcoxy en C₁-C₄, un groupe amino, un groupe acyl (C₂-C₄)-oxy, un groupe acyl (C₂-C₄)-amino, un groupe triméthylsilyloxy ou un groupe hydroxyalkyl (C₂-C₄)-amino ;
- R⁵ et R⁶ ne pouvant désigner simultanément un radical alcoxy en C₁-C₄;
- R⁵ et R⁶ pouvant également former, conjointement avec les atomes de carbone auxquels ils sont rattachés, un cycle carbonyldioxy ;
- au moins l'un des radicaux R⁴ à R⁷ représente un groupement OZ ou NHR⁸, l'un au plus des radicaux R⁴ à R⁷ représentant NHR⁸, et deux au plus des radicaux R⁴ à R⁷ représentant OZ et, dans le cas où Z représente un atome d'hydrogène, les deux groupes OH sont dans les positions 5 et 6 ; et au moins l'un des radicaux R⁴ à R⁷ représente un atome d'hydrogène, et dans le cas où un seul de ces radicaux R⁴ à R⁷ représente un atome d'hydrogène, alors un seul radical parmi R⁴ à R⁷ représente alors NHR⁸ ou OZ, les autres radicaux représentant un groupe alkyle en C₁-C₄ ;
- le radical R⁸ du groupement NHR⁸ désignant un atome d'hydrogène, un groupe acyle en C₂-C₄ ou hydroxyalkyle en C₂-C₄, et le radical Z du groupement OZ désignant un atome d'hydrogène, un groupe acyle en C₂-C₁₄, un groupe alkyle en C₁-C₄, ou un groupe triméthylsilyle,
   et leurs sels de métaux alcalins, alcalino-terreux, d'ammonium ou d'amines.

Les composés indoliques de formule (I) ci-dessus sont choisis, parmi le 4-hydroxyindole, le 5-hydroxyindole, le 6-hydroxyindole, le 7-hydroxyindole, le 4-hydroxy 5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxy, le 6-hydroxyindole, N-méthylindole, 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylthdole, le 6-hydroxy 2-éthoxycarbonyl 5-méthoxyindole, le 6-N,β-hydroxyéthylaminoindole, le 4-aminoindole, le 5-aminoindole, le 6-aminoindole, le 7-aminoindole, le N-méthyl 6-β-hydroxyéthylominoindole, le 6-amino 2,3-diméthylindole, le 6-amino 2,3,4,5-tétraméthylindole, le 6-amino2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-triméthylsilyloxyindole, l'ester phosphorique du 5,6-dihydroxyindole, le 5,6-dibenzyloxyindole, et les sels d'addition de ces composés.

Les composés indoliques particulièrement préférés sont : le 5,6-dihydroxyindole, le 6-hydroxyindole, le 7-hydroxyindole, le bromhydrate de 2-méthyl 5,6-dihydroxyindole le 7-aminoindole, le 3-méthyl 5,6-dihydroxyindole, le 4-hydroxy 5-méthoxyindole, 2,3-diméthyl 5-méthoxy 6-hydroxyindole

La polymérisation oxydante des composés de formule (I) peut s'effectuer en milieu aqueux, eau/solvant (s), ou solvant (s) à l'air, en présence ou non d'un agent alcalin et/ ou d'un agent oxydant tel que le peroxyde d'hydrogène de préférence en présence d'un agent alcalin tel que l'ammoniaque ou en présence d'un ion iodure, l'iodure étant de préférence un iodure de métal alcalin, alcalino-terreux ou d'ammonium.

L'oxydation du composé de formule (I) peut également être effectuée en utilisant l'acide periodique et ses sels hydrosolubles et dérivés, les permanganates et bichromates, tels que de sodium ou de potassium, l'hypochlorite de sodium, le persulfate d'ammonium, le nitrite de sodium et des oxydants organiques choisis parmi les ortho- et parabenzoquinones, les ortho-et parabenzoquinones mono- ou diimines, les 1,2 et 1,4-naptoquinones, les 1,2-et 1,4-naphtoquinones mono-ou diimines telles que décrites dans la demande EP-A-O376 776. Le sel d'acide periodique préféré est le periodate de sodium.

Il est possible d'activer les agents oxydants par un modificateur de pH.

Conformément à l'invention, le procédé de polymérisation oxydante préféré met en oeuvre le peroxyde d'hydrogène en présence d'ammoniaque. Cette réaction d'oxydation s'effectue généralement à une température ambiante de l'ordre de 20°C à 100°C et de préférence 60°C à 90°C.

La réalisation de la polymérisation oxydante s'effectue de préférence en introduisant le composé indolique de formule (I) dans un milieu aqueux, ou dans un mélange d'eau et d'un ou plusieurs solvants pouvant contenir jusqu'à 95 % de solvant ou encore dans un ou plusieurs solvant (s) anhydre (s), c'est-à-dire contenant moins de 1 % d'eau.

Parmi les solvants utilisables on peut citer les alcools inférieurs en C₁-C₄ tels que l'alcool éthylique, l'alcool propylique ou isopropylique, l'alcool tert.-butylique, les alkylèneglycols, tels que l'éthylèneglycol, le propylène glycol, les alkylèthers d'alkylèneglycols, tels que les éthers monométhylique, monoéthylique ou monobutylique de l'éthylèneglycol, les monométhyléthers du propylèneglycol et du dipropylèneglycol, et des esters tels que le lactate de méthyle. Le milieu solvant préféré est un milieu hydroalcoolique contenant de 1 à 10 % d'alcool éthylique.
Suivant les procédés on laisse l'oxydant et le composé indolique de formule (I) en contact pendant quelques minutes à quelques jours.

Les agents alcalinisants sont de préférence choisis parmi l'hydroxyde de sodium, les carbonates alcalins ou l'ammoniaque, dans des proportions comprises entre 5 X 10⁻⁴ % à 10 % en poids par rapport au poids de la composition soumise à l'oxydation.

Lorsqu'on utilise un iodure en présence de peroxyde d'hydrogène on utilise de préférence un iodure de sodium ou de potassium à une concentration comprise entre 1 et 6 %.

Le pigment coloré résultant de la polymérisation oxydante est obtenu sous forme insoluble. Il est isolé par filtration ou centrifugation. Afin d'éliminer les traces de composé de formule (I) n'ayant pas réagi, on peut rincer le pigment à l'eau avant ou après filtration ou centrifugation.

Dans le cas où l'on met en oeuvre un procédé de polymérisation oxydante à l'air, il est également possible d'isoler le pigment par lyophilisation.

Afin d'obtenir un pigment homogène et de granulométrie suffisamment fine, il est possible de traiter le produit obtenu à la suite de la polymérisation oxydante par des systèmes de broyages classiques par voie sèche ou humide. On peut également utiliser un procédé de micronisation.

La granulométrie du pigment final est généralement telle que 90 % des particules aient un diamètre inférieur à 100 µm et de préférence à 50 µm. Le diamètre moyen des particules est de préférence inférieur à 50 µm et en particulier inférieur à 20 µm.

Les pigments insolubles ainsi obtenus sont utilisés dans des compositions de coloration des fibres kératiniques à des concentrations pondérales généralement comprises entre 0,05 et 10 % et de préférence entre 0.2 et 2 % en poids par rapport au poids total de la composition.

Ces compositions de coloration temporaire sont généralement aqueuses. Elles peuvent cependant contenir un ou plusieurs solvants choisis parmi les alcools inférieurs comme par exemple l'isopropanol ou l'éthanol, les polyols comme le propylèneglycol ou le glycérol, les éthers de glycols comme par exemple les monométhyl, monoéthyl ou monobutyléthers de l'éthylèneglycol, les monométhyléthers du propylèneglycol, ou du dipropylèneglycol ou du tripropylèneglycol ou des esters comme le lactate de métyle.

Ces solvants sont généralement présents dans des proportions en poids comprises entre 1 et 75 % et de préférence entre 2 et 50 % par rapport au poids total de la composition.

Selon une forme de réalisation préférée les compositions conformes à l'invention peuvent contenir un agent de suspension des pigments ou un agent épaississant afin de faciliter la répartition homogène du pigment insoluble dans la composition et sur les fibres kératiniques.

Ces agents de suspension ou épaississants peuvent être choisis parmi les dérivés de cellulose tels que par exemple la carboxyméthylcellulose, l'hydroxyéthyl ou l'hydroxypropylcellulose, les biopolysaccharides tels que les gommes de xanthane ou les scléroglucanes, la gomme de guar ou des épaississants minéraux comme les dérivés d'argiles ou les silices.

On peut également utiliser les produits résultant de l'interaction ionique d'un polymère cationique constitué par un copolymère de cellulose ou d'un dérivé de cellulose greffés par un sel de monomère hydrosoluble d'ammonium quaternaire et d'un polymère anionique carboxylique tels que décrits dans le brevet français FR - 2 598 611. On utilise de préférence le produit d'interaction ionique d'un copolymère d'hydroxyéthylcellulose greffé par voie radicalaire par du chlorure de diallyldiméthylammonium tel que le polymère commercialisé sous la dénomination CELQUAT L 200 par la société National Starch avec, soit des copolymères d'éthylène et d'anhydride maléique tels que les produits vendus sous la dénomination EMA 31 par la société MONSANTO, soit des copolymères 50/50 d'acide méthacrylique et de méthacrylate de méthyle.

Un autre produit de ce type utilisable est le produit résultant de l'interaction ionique du copolymère d'hydroxyéthyl cellulose greffé par voie radicalaire par du chlorure de diallyldiméthylammonium avec un polymère anionique carboxylique réticulé tel que les copolymères de l'acide méthacrylique et de l'acrylate d'éthyle réticulés vendus sous la dénomination VISCOATEX 538 ou 46 par la société COATEX.

On peut également utiliser dans ce but des dispersions aqueuses de copolymère d'acrylate d'ammonium / acrylamide réticulé par un agent de réticulation à polyinsaturation olèfinique dispersé dans une émulsion eau-dans-huile comprenant la paraffine et un mélange de stéarate de sorbitan et de dérivé éthoxylé hydrophile et plus particulièrement l'émulsion commercialisée sous la dénomination de PAS 5161 par la société HOECHST, et comprenant le copolymère acrylate d'ammonium / acrylamide (95/5 en poids) . Cette composition commerciale contient 30 % en poids de ce copolymère, 25 % en poids de paraffine, 4 % de mélange sorbitan et de dérivé éthoxylé hydrophile et 41 % d'eau.

Un autre agent épaisissant et/ou de mise en suspension des pigment insolubles utilisés conformément à l'invention est constitué par des copolymères de méthylvinyléther et d'anhydride maléique réticulés ou non, tel que le produit vendu sous la dénomination GANTREZ ACV neutralisé ou des acides polyacryliques réticulés tels que les produits vendus sous la dénomination CARBOPOL par la société GOODRICH ou des copolymères d'acide acrylique et de méthacrylate ou acrylate d'alkyle à longue chaîne en (C10-C30) réticulés, vendus respectivement sous les dénominations CARBOPOL 1342 et PEMULEN TR-1 et TR-2 par la société GOODRICH. On utilise de préférence des copolymères réticulés.
Ces agents épaisissants sont utilisés généralement dans les compositions de coloration temporaire conformes à l'invention, dans des proportions comprises entre 0,05 et 10 % en poids par rapport au poids total des compositions.

Dans une forme de réalisation préférée de l'invention on utilise dans les compositions contenant le pigment insoluble issu de la polymérisation oxydante des composés de formule (I) , un latex filmogène qui permet d'obtenir des compositons de coloration temporaire présentant une meilleure résistance au brossage et conférant aux fibres traitées une brillance supérieure. Ces latex peuvent être présents dans des proportions comprises entre 0,1 et 5 % en poids par rapport au poids total de la composition.

On appelle un latex filmogène un latex dont 100ml d'une solution à 8 g/100 ml déposés dans une matrice rigoureusement horizontale de 65 cm² de surface, laisse apparaître à l'oeil nu après 15 heures de séchage à la température ambiante, une fine pellicule homogène uniforme sur toute la surface de la matrice. Ce latex est choisi notamment parmi :
- les homopolymères et des copolymères d'acétate de vinyle, tels que : le polyacétate de vinyle comme les produits vendus sous les dénominations, "Emulsion RHODOPAS AO12P" "Emulsion RHODOPAS AO13P" par la société RHONE POULENC; les copolymères d'acétate de vinyle et d'éthylène, tels que les produits vendus sous les dénominations "APPRETAN MB", "APPRETAN EM", "APPRETAN TV" par la société HOECHST.
- les homopolymères ou les copolymères dérivés d'acide acrylique, tels que les produits vendus sous les dénominations "PRIMAL AC-33, "PRIMAL K-3", "PRIMAL TR-93, "PRIMAL HA-8", "PRIMAL E-358", commercialisés par la société RHOM & HAAS ;
- les polyuréthanes, tels que ceux vendus sous la dénomination "WITCOBOND 160", "WITCOBOND 170", par la société WITCO,
- les copolymères butadiène/styrène carboxylés ou non, tels que les produits vendus sous les dénominations "Emulsion RHODOPAS SB02, "Emulsion RHODOPAS ST246, "Emulsion RHODOPAS SB153", "Emulsion RHODOPAS GB012" par la société RHONE POULENC;
- les copolymères butadiène/ acrylonitrile, carboxylés ou non, tels que les produits vendus sous les dénominations "HYCAR 1562" par la société GOODRICH et "CHEMIGUM L6271" par la société GOODYEAR ;
- les copolymères styrène/esters acryliques tels que le produit vendu sous la dénomination "APPRETAN V3749" par la société HOECHST;
- les copolymères acétate de vinyle/ esters acryliques tel que le produit vendu sous la dénomination"Emulsion RHODOPAS AD 310";
- les terpolymères styrène / butadiene / vinylpyridine tel que le produit vendu sous la dénomination HYCAR 2508 par la société GOODRICH;
- les copolymères du chlorure de vinyle ou du chlorure de vinylidène tels que les produits vendus sous les dénominations GEON 351 ou 577 par la société GOODRICH.

Une autre forme de réalisation préférée de l'invention consiste à utiliser dans les compositions contenant le pigment conforme à l'invention, un copolymère acrylates tertio-octylpropenamide tel que le produit vendu sous la dénomination DERMACRYL 79 par la société NATIONAL STARCH. Ces compositions ont une rémanence à l'eau améliorée.

Les compositions utilisées pour la coloration temporaire des fibres kératiniques conformes à l'invention peuvent se présenter sous des formes diverses telles que de liquides plus ou moins épaissis, de crèmes, de gels, ou de sticks ; on préfère cependant utiliser les gels.

Les gels contiennent dans les milieux aqueux, eau/ solvant (s) ou solvant (s) définis ci-dessus au moins un agent de suspension ou épaisissant décrit ci-dessus.

Les compositions destinées à être utilisées pour la coloration temporaire des fibres kératiniques selon l'invention peuvent contenir en outre divers adjuvants habituellement utilisés dans les compositions capillaires. Parmi ces adjuvants on peut citer les silicones insolubles ou solubles, volatiles ou non, sous forme d'huiles, de gommes, de résines ou de poudres, des polymères non ioniques anioniques, cationiques ou amphotères ; des protéines quaternisées ou non ; des filtres solaire ; des agents tensioactifs ; des agents antimousse ; des hydratants ; des humectants ; des émollients ; des huiles végétales ou synthétiques ; des agents conservateurs, des agents séquestrants ; des agents antioxydants ; des agents nacrants et des parfums ; des agents alcalinisants ou acidifiants ; d'autres pigments tels que ceux décrits dans l'ouvrage COLOUR INDEX à l'exclusion des nanopigments d'oxyde de titane, de zinc, de cenium et zirconium ainsi que d'autres colorants directs généralement connus en coloration temporaire.

Les pigments insolubles résultant de la polymérisation oxydante des dérivés indoliques de formule (I) définis ci-dessus sont utilisés de préférence pour la teinture de cheveux. Ils sont utilisés en particulier pour estomper et embellir les cheveux blancs. L'application est suivie d'un séchage des cheveux et d'un coiffage.

Ces pigments peuvent également être utilisés dans les compositions destinées à être appliquées sur les poils tels que les cils notamment dans les compositions de mascara.

Les exemples suivants sont destinés dans l'invention sans pour autant présenter un caractère limitatif.

### EXEMPLE 1

On prépare un gel capillaire de composition suivante :

| | |
|---|---|
| - Acide polyacrylique réticulé vendu sous la dénomination de CARBOPOL 940 (PM 4.000.000) par la société GOODRICH | 0,3 g |
| - Copolymère (65/35) de polyvinylpyrrolidone et d'acétate de vinyle vendu sous la dénomination PVP/VA S 630 par la société GAF | 4 g |
| Alcool éthylique | 17 g |
| Pigment préparé selon l'exemple de préparation 1. | 1 g |
| Triéthanolamine qs pH 7,5 | |
| Eau qsp | 100 g |

Le gel est utilisé pour la coloration temporaire des cheveux blancs. Les cheveux sont alors colorés en gris naturel.
On peut remplacer le pigment obtenu selon l'exemple de préparation 1 par la même quantité de pigment obtenu selon l'exemple de préparation 2, 3 ou 4.

### EXEMPLE 2

On prépare un gel capillaire de composition suivante :

| | |
|---|---|
| - Emulsion de copolymère acrylate d'ammonium/acrylamide réticulé vendue sous la dénomination PAS 5161 par la société HOECHST (à 30 % de MA en copolymère) | 0,6 gMA |
| - Copolymère (65/35) de polyvinylpyrrolidone et d'acétate de vinyle vendu sous la dénomination PVP/VA S 630 par la société GAF | 4 g |
| Pigment préparé selon l'exemple de préparation 1 | 1 g |
| Triéthanolamine qs pH 7,5 | |
| Eau qsp | 100 g |

Le gel est appliqué sur cheveux blancs et confère une coloration temporaire éliminable au premier shampooing.

### EXEMPLE 3

On prépare un gel capillaire de composition suivante :

| | |
|---|---|
| - Copolymère d'acide acrylique et d'acrylate d'alkyle (C₁₀-C₃₀) réticulé vendu sous la dénomination PEMULEN TR1 par la société GOODRICH (totalement neutralisé par la triéthanolomine) (avant neutralisation ) | 0,5 g |
| - Latex acrylique aqueux vendu à 40 % de MA sous la dénomination PRIMAL K3 par la société RHOM & HAAS | 1,3 gMA |
| - Copolymère (65/35) de polyvinylpyrrolidone et d'acétate de vinyle vendu sous la dénomination PVP/VA S 630 par la société GAF | 2 g |
| Pigment préparé selon l'exemple de préparation 1 | 1 g |
| Triéthanolamine qs pH 7,5 | |
| Conservateur qs | |
| Eau qsp | 100 g |

Le gel est appliqué sur cheveux blancs et confère une coloration temporaire éliminable au premier shampooing.

### EXEMPLE 4

On prépare un gel capillaire de composition suivante :

| | |
|---|---|
| - Copolymère d'acide acrylique et d'acrylate d'alkyle (C₁₀-C₃₀) réticulé vendu sous la dénomination PEMULEN TR1 par la société GOODRICH totalement neutralisé par de la triéthanolamine (avant neutralisation) | 0,6 g |
| - Copolymère (65/35) de polyvinylpyrrolidone et d'acétate de vinyle vendu sous la dénomination PVP/VA S 630 par la société GAF | 2 g |
| Pigment préparé selon l'exemple de préparation 1 | 1 g |
| Triéthanolamine qs pH 7,5 | |
| Conservater qs | |
| Eau qsp | 100 g |

Le gel est appliqué sur cheveux blancs et confère une coloration temporaire éliminable au premier shampooing.

### EXEMPLE 5

On prépare un gel capillaire de composition suivante :

| | |
|---|---|
| - Copolymère d'acide méthacrylique et d'acrylate d'éthyle réticulé vendu en dispersion aqueuse à 38 % de MA sous la dénomination VISCOATEX 538 par la société COATEX | 2,28 gMA |
| - Copolymère d'hydroxyéthylcellulose greffé par voie radicalaire par du chlorure de diallyl diméthyl ammonium, vendu par la société NATIONAL STARCH sous la dénominatoin CELQUAT L 200 | 1 g |
| Silicone aminée vendue en émulsion cationique à 35 % de MA sous la dénomination Emulsion DC 929 par la société DOW CORNING | 0,3 gMA |
| Pigment préparé selon l'exemple de préparation 1 | 1 g |
| 2-Amino- 2-méthyl-1-propanol qs pH | 7,5 |
| Conservateur, parfum qs | |
| Eau qsp | 100 g |

Le gel est appliqué sur cheveux blancs et confère une coloration temporaire éliminable au premier shampooing.

### EXEMPLE 6

On prépare un mascara sous forme de gel ayant la composition suivante :

| | |
|---|---|
| - Pigment micronisé issu de la polymérisation de 5,6-dihydroyindole, selon le procédé décrit dans l'exemple 1. | 10 g |
| - Carbopol 934 vendu par la société GOODRICH | 0.80 g |
| Triéthanolamine à 99 % | 1,04 g MA |
| Polyvinylpyrrolidone | 0,45 g |
| Propylène glycol | 3.50 g |
| Copolymère d'alcool vinylique/acétate de vinyle | 2,09 g |
| D- Panthénol | 1 g |
| Conservateurs q.s | |
| Eau q.s.p | 100 g |

### EXEMPLE 7

| | |
|---|---|
| - Pigment issu de la polymérisation du 5,6-dihydroxyindole, préparé selon l'exemple 1 | 1 g |
| - Copolymère d'acide méthacrylique et d'acrylate d'éthyle réticulé vendu en dispersion aqueuse à 38 % de MA sous la dénomination VISCOATEX 538 par la société COATEX | 1,5 g MA |
| - Copolymère d'hydroxyéthylcellulose greffé par voie radicalaire par du chlorure de diallyl diméthylammonium, vendu par la société NATIONAL STARCH sous la dénomination CELQUAT L 200, mis en solution aqueuse à 5 % | 1,5 g MA |
| Alcool éthylique | 10 g |
| - Copolymère acrylates / t-octylpropenamide vendu sous la dénomination DERMACRYL 79 par la société NATIONAL STARCH | 1,5 g |
| Stéaryldiméthylamine | 3,75 g |
| 2-amino 2-méthyl 1-propanol qs pH : 7,5 | |
| Conservateur, parfum, antimousse qs | |
| Eau qsp | 100 g |

### EXEMPLE DE PREPARATION 1

On dissout, dans 5 litres d'eau, 500g (3,26 moles) de 5,6-dihydroxyindole. On ajoute 20 ml d'ammoniaque en solution à 20 % et on porte le tout à 80° C. On additionne goutte-à-goute à cette température, le mélange constitué de 750 ml d'eau et 750 g d'une solution de peroxyde d'hydrogène à 50 %, en deux heures. Le chauffage est maintenu deux heures supplémentaires après la fin de l'addition.

Le précipité noir est essoré, lavé à l'eau puis à l'alcool. Le produit est séché sous vide. On isole 508 g de pigment.

Ce pigment peut-être micronisé.

Dans une variante de ce procédé le précipité avant lavage à l'alcool et sèchage, est passé en milieu humide dans un broyeur à billes. Le pigment a une granulométrie moyenne inférieure à 20 µm

### EXEMPLE DE PREPARATION 2

On dissout à 20° C, 90 g (0,6 mole) de 5,6-dihydroxyindole dans 6 litres d'eau.

Sous agitation mécanique on injecte de l'air comprimé pendant 168 heures.

On centrifuge le milieu réactionnel.

Le précipité est lavé à l'eau puis essoré.

On obtient 350 g de pigment noir, humide, titrant 27,5 % en matière sèche.

### EXEMPLE DE PREPARATION 3

On dissous 50 g (0,32 mole) de 5,6-dihydroxyindole dans 500 ml d'eau et on porte à 80° C.

On additionne en 4 heures le mélange constitué par 49 g de solution de peroxyde d'hydrogène à 50 % et de 39 g d'eau.

L'addition terminée, on maintient l'agitation deux heures supplémentaires.

Le milieu réactionnel est refroidi à température ambiante et le précipité formé est essoré, lavé à l'eau puis séché sous vide.

On obtient 49,8 g de pigment.

### EXEMPLE DE PREPARATION 4

On dissout 6 g (0,04 mole) de 5,6-dihydroxyindole dans 180 ml d'eau additionnée de 20 ml d'éthanol et 30 ml d'eau oxygénée à 20 volumes.

On ajoute à ce mélange à température ambiante une solution de 2,5 g (1,5. 10⁻² mole) d'iodure de potassium dissous dans 25 ml d'eau en 30 minutes.

L'agitation est maintenue 3 heures supplémentaires en fin d'addition.

Le précipité est essoré, lavé à l'eau puis à l'alcool.

On sèche le produit sous vide.

On obtient 6 g de pigment.

## Revendications

1. Utilisation pour la teinture temporaire des fibres kératiniques du pigment insoluble, isolé obtenu après polymérisation oxydante d'un composé indolique répondant à la formule (I) 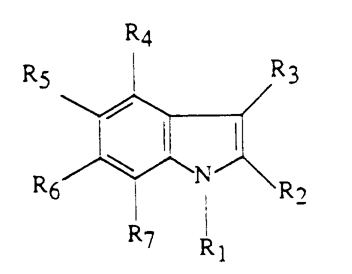 dans laquelle :
- R¹ et R³ représentent, indépendamment l'un de l'autre, un atome d'hydrogène ou un groupe alkyle en C₁-C₄ ;
- R² représente un atome d'hydrogène, un groupe alkyle en C₁-C₄, un groupe carboxyle ou un groupe alcoxy (C₁-C₄)-carbonyle ;
- R⁴ et R⁷ représentent, indépendamment l'un de l'autre, un atome d'hydrogène, un groupe hydroxy, un groupe alkyle en C₁-C₄, un groupe amino, un groupe alcoxy en C₁-C₄, un groupe acyl (C₂-C₄)-oxy ou un groupe acyl (C₂-C₄)-amino;
- R⁵ représente un atome d'hydrogène, un groupe hydroxy, un groupe alcoxy en C₁-C₄, un groupe alkyle en C₁-C₄, un atome d'halogène, un groupe amino, un groupe acyl (C₂-C₁₄)-oxy, un groupe acyl (C₂-C₄)-amino ou un groupe triméthylsilyloxy ;
- R⁶ représente un atome d'hydrogène, un groupe hydroxy, un groupe alcoxy en C₁-C₄, un groupe amino, un groupe acyl (C₂-C₄)-oxy, un groupe acyl (C₂-C₄)-amino, un groupe triméthylsilyloxy ou un groupe hydroxyalkyl (C₂-C₄)-amino;
- R⁵ et R⁶ ne pouvant désigner simultanément un radical alcoxy en C₁-C₄ ;
- R⁵ et R⁶ pouvant également former, conjointement avec les atomes de carbone auxquels ils sont rattachés, un cycle carbonyldioxy ;
- au moins l'un des radicaux R⁴ à R⁷ représente un groupement OZ ou NHR⁸, l'un au plus des radicaux R⁴ à R⁷ représentant NHR⁸, et deux au plus des radicaux R⁴ à R⁷ représentant OZ et, dans le cas où Z représente un atome d'hydrogène, les deux groupes OH sont dans les positions 5 et 6 ; et au moins l'un des radicaux R⁴ à R⁷ représente un atome d'hydrogène, et dans le cas où un seul de ces radicaux R⁴ à R⁷ représente un atome d'hydrogène, alors un seul radical parmi R₄ à R₇ représente NHR⁸ ou OZ, les autres radicaux représentant un groupe alkyle en C₁-C₄ :
- le radical R⁸ du groupement NHR⁸ désignant un atome d'hydrogène, un groupe acyle en C₂-C₄ ou hydroxyalkyle en C₂-C₄, et le radical Z du groupement OZ désignant un atome d'hydrogène, un groupe acyle en C₂-C₁₄, un groupe alkyle en C₁-C₄, ou un groupe triméthylsilyle,
et leurs sels de métaux alcalins, alcalino-terreux, d'ammonium ou d'amines.

2. Utilisation selon la revendication 1 caractérisée par le fait que le composé indolique de formule (I) est choisi parmi : le 4-hydroxyindole, le 5-hydroxyindole, le 6-hydroxyindole, le 7-hyroxyindole, le 4-hydroxy-5-méthoxyindole le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihyroxyindole, le N-méthyl 5,6-dihydroxyindole, le 2-méthyl 5,6-dihydroxrin-dole, le 3-méthyl 5,6-dihydroxyindole, le 2,3-diméthyl 5,6-dihydroxyindole, le 2-carboxy 5,6-hyroxyindole, le 4-hydroxy 5-méthylindole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N,β-hydroxyéthylaminoindole, le 4-aminoindole, le 5-aminoindole, le 6-aminoindole, le 7-aminoindole, le N-méthyl 6-β-hydroxyéthylaminoindole, le 6-amino 2,3-diméthylindole, le 6-amino 2,3,4,5-tétraméthylindole, le 6-amino2,3,4-triméthylindole, le 6-amino 2,3,5-triméthylindole, le 6-amino 2,3,6-triméthylindole, le 5,6-diacétoxyindole. le 5-méthoxy 6-acétoxyindole, le 5,6-triméthylsilyloxyindole, l'ester phosphorique du 5,6-dihydroxyindole, le 5,6-dibenzyloxyindole, et les sels d'addition de ces composés.

3. Utilisation selon la revendication 1, caractérisée par le fait que le composé indolique est choisi parmi : le 5,6-dihydroxyindole, le 6-hydroxyindole, le 7-hydroxyindole, le bromhydrate de 2-méthyl 5,6-dihydroxyindole, le 7-aminoindole, le 3-méthyl 5,6-dihydroxyindole, le 4-hydroxy 5-méthoxyindole, 2,3-diméthyl 5-méthoxy 6-hyroxyindole.

4. Utilisation selon l'une quelconque des revendications 1 à 3, caractérisée par le fait que la polymérisation oxydante pour former le pigment est effectuée en milieu aqueux, eau/solvant (s) ou solvant (s) à l'air en présence ou non d'un agent alcalin et/ ou d'un agent oxydant.

5. Utilisation selon la revendication 4 caractérisée par le fait que l'agent oxydant est choisi parmi le peroxyde hydrogène, le peroxyde d'hydrogène utilisé en présence d'un agent alcalin, le peroxyde d'hydrogène utilisé en présence d'un ion iodure, l'acide periodique et ses sels hydrosolubles et dérivés, les permanganates, les bichromates, l'hypochlorite de sodium, le persulfate d'ammonium, le nitrite de sodium.

6. Utilisation selon la revendication 4, caractérisée en par le fait que l'agent oxydant est choisi parmi les ortho- et parabenzoquinones, les ortho-ou parabenzoquinones mono- ou diimines, les 1,2- et 1,4-naphtoquinone, les 1,2- et 1,4-naphtoquinone mono- ou diimines.

7. Utilisation selon l'une queconque des revendications 1 à 4, caractérisée par le fait que le pigment résulte de la polymérisation oxydante effectuée en milieu aqueux et/ou eau/solvant (s) par du peroxyde d'hydrogène en présence d'ammoniaque.

8. Utilisation selon l'une quelconque des revendications 1 à 7 caractérisée par le fait que la granulométrie des particules de pigment est telle que 90% de particules aient un diamètre inférieur à 100 µm et de préférence inférieur à 50 µm.

9. Utilisation selon l'une quelconque des revendications 1 à 8 caractérisée par le fait que le pigment résultant de la polymérisation oxydante des composés indoliques de formule (I) a une granulométrie telle que le diamètre moyen des particules est inférieur à 50 µm et de préférence inférieur à 20 µm.

10. Utilisation selon l'une quelconque des revendications 1 à 9 caractérisée par le fait que le pigment insoluble résultant de la polymérisation oxydante de dérivés indolique de formule (I) est utilisé dans une composition, comprenant un milieu approprié pour l'application du pigment sur les fibres, en une proportion comprise autre 0,05 et 10 % de préférence 0,2 et 2% en poids par rapport au poids total de la composition.

11. Utilisation selon la revendication 10 caractérisée par le fait que le milieu approprié pour la coloration temporaire est un milieu aqueux pouvant contenir un ou plusieurs solvants choisis parmi les alcools inférieurs, les polyols, les éthers de glycols, les esters ou leurs mélanges.

12. Utilisation selon les revendications 10 ou 11 caractérisée par le fait que la composition contient un agent de mise en suspension ou épaisissant permettant de répartir de façon homogène les pigments insolubles dans la composition et sur les fibres traitées.

13. Utilisation selon la revendication 12 caractérisée par le fait que l'agent de suspension ou épaississant est choisi parmi les dérivés de cellulose, les biopolysaccharides, la gomme de guar ou des épaisissants minéraux.

14. Utilisation selon l'une des revendications 10 à 13 caractérisée par le fait que le milieu approprié pour la coloration temporaire contient un latex filmogène.

15. Composition destinée à être utilisée pour la coloration temporaire des fibres kératiniques caractérisée par le fait qu'elle contient dans un milieu approprié pour la coloration temporaire au moins un pigment insoluble isolé résultant de la polymérisation oxydante de dérivés indoliques répondant à la formule (I) 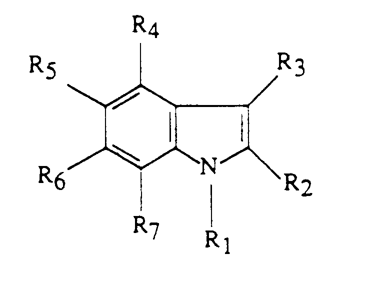 dans laquelle :
- R¹ et R³ représentent, indépendamment l'un de l'autre, un atome d'hydrogène ou un groupe alkyle en C₁-C₄ ;
- R² représente un atome d'hydrogène, un groupe alkyle en C₁-C₄, un groupe carboxyle ou un groupe alcoxy (C₁-C₄)-carbonyle ;
- R⁴ et R⁷ représentent, indépendamment l'un de l'autre, un atome d'hydrogène, un groupe hydroxy, un groupe alkyle en C₁-C₄, un groupe amino, un groupe alcoxy en C₁-C₄, un groupe acyl (C₂-C₄)-oxy ou un groupe acyl (C₂-C₄)-amino :
- R⁵ représente un atome d'hydrogène, un groupe hydroxy, un groupe alcoxy en C₁-C₄, un groupe alkyle en C₁-C₄, un atome d'halogène, un groupe amino, un groupe acyl (C₂-C₁₄)-oxy, un groupe acyl (C₂-C₄)-amino ou un groupe triméthylsilyloxy ;
- R⁶ représente un atome d'hydrogène, un groupe hydroxy, un groupe alcoxy en C₁-C₄, un groupe amino, un groupe acyl (C₂-C₄)-oxy, un groupe acyl (C₂-C₄)-amino, un groupe triméthylsilyloxy ou un groupe hydroxyalkyl (C₂-C₄)-amino ;
- R⁵ et R⁶ ne pouvant désigner simultanément un radical alcoxy en C₁-C₄ ;
- R⁵ et R⁶ pouvant également former, conjointement avec les atomes de carbone auxquels ils sont rattachés, un cycle carbonyldioxy ;
- au moins l'un des radicaux R⁴ à R⁷ représente un groupement OZ ou NHR⁸, l'un au plus des radicaux R⁴ à R⁷ représentant NHR⁸ et deux au plus des radicaux R⁴ à R⁷ représentant OZ et, dans le cas où Z représente un atome d'hydrogène, les deux groupes OH sont dans les positions 5 et 6 : et au moins l'un des radicaux R⁴ à R⁷ représente un atome d'hydrogène, et dans le cas où un seul de ces radicaux R⁴ et R⁷ représente un atome d'hydrogène, alors un seul radical parmi R⁴ à R⁷ représente NHR⁸ ou OZ, les autres radicaux représentent un groupe alkyle en C₁-C₄ ;
- le radical R8 du groupement NHR⁸ désignant un atome d'hydrogène, un groupe acyle en C₂-C₄ ou hydroxyalkyle en C₂-C₄, et le radical Z du groupement OZ désignant un atome d'hydrogène, un groupe acyle en C₂-C₁₄, un groupe alkyle en C₁-C₄, ou un groupe triméthylsilyle,
et leurs sels de métaux alcalins, alcalino-terreux, d'ammonium ou d'amines
et au moins un agent de mise en suspension ou épaisissant choisi parmi la carboxyméthylcellulose, l'hydroxyéthyl cellulose, l'hydroxypropylcellulose ; les gommes de xanthane ou les scéroglucanes, la gomme de guar : les épaississants minéraux : les produits réticulés ou non résultant de l'interaction ionique d'un polymère cationique constitué par un copolymère de cellulose ou d'un dérivé de cellulose greffés par un sel de monomère hydrosoluble d'ammmonium quaternaire et d'un polymère anionique carboxylique ; des dispersions aqueuses de copolymères d'acrylate d'ammonium/acrylamide réticulés par un agent de réticulation à polymérisation oléfinique dispersés dans une émulsion eau-dans-huile ; les copolymères de méthylvinyléther et d'anhydride maléique réticulés ou non ; des acides polyacryliques réticulés ; les copolymères d'acide acrylique et de méthacrylate ou d'acrylate d'alkyle à longue chaîne (C10-C30) réticulés.

16. Composition selon la revendication 15 caractérisée par le fait que l'agent de mise en suspension ou épaississant est utilisé dans des proportions comprises entre 0,05 et 10% en poids par rapport au poids total de la composition.

17. Composition destinée à être utilisée pour la coloration temporaire des fibres kératiniques caractérisée isolé par le fait qu'elle contient dans un milieu approprié pour la coloration temporaire au moins un pigment insoluble isolé issu de la polymérisation oxydante du composé de formule (I) tel que défini dans la revendication 1, et un latex filmogène.

18. Composition selon la revendication 17 caractérisée par le fait que le latex filmogène est choisi parmi les homopolymères et les polymères d'acétate de vinyle; les homopolymères et les copolymères dérivés d'acide acrylique ; les polyuréthanes ; les copolymères butadiène/styrène carboxylés ou non ; les copolymères butadiène/acrylonitrile carboxylés ou non ; les copolymères styrène/esters acryliques ; les copolymères acétate de vinyle/esters acryliques ; les terpolymères styrène / butadiène / vinylpyridine ; les copolymères du chlorure de vinyle ou du chlorure de vinylidène.

19. Utilisation selon l'une quelconque des revendications 1 à 14 caractérisée par le fait que le pigment insoluble résultant de la polymérisation oxydante des composés de formule (I) est mis en oeuvre sous forme d'une composition telle que définie dans l'une quelconque des revendications 15 à 18.

20. Utilisation selon l'une quelconque des revendications 1à 14 et 19 caractérisée par le fait que la composition de coloration temporaire contient en outre des silicones insolubles ou solubles, volatiles ou non, sous forme d'huiles. de gommes, de résines ou de poudres : des polymères non ioniques, anioniques, cationiques ou amphotères ; des protéines quaternisées ou non ; des filtres solaires ; des agents tensioactifs ; des agents antimousse ; des hydratants : des humectants : des émollients : des huiles végétales ou synthétiques : des conservateurs : des agents séquestrants : des agents antioxydants : des agents nacrants : des parfums : des agents alcanisants ou acidifiants : des pigments : des colorants directs.

21. Procédé de coloration temporaire des fibres kératiniques caractérisé par le fait que l'on applique sur ces fibres une composition contenant, dans un milieu approprié pour la teinture de ces fibres, un pigment insoluble isolé résultant de la polymérisation oxydante des composés indoliques tels que définis dans l'une quelconque des revendications 1 à 3.

## Claims

1. Use, for the temporary dyeing of keratinous fibres, of the isolated, insoluble pigment obtained after oxidative polymerization of an indole compound corresponding to the formula (I) 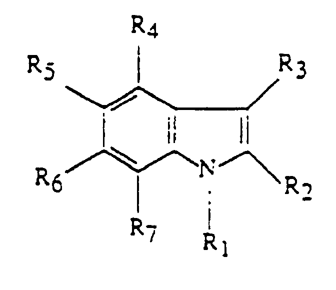 in which:
- R¹ and R³ represent, independently of one another, a hydrogen atom or a C₁-C₄ alkyl group;
- R² represents a hydrogen atom, a C₁-C₄ alkyl group, a carboxyl group or a (C₁-C₄ alkoxy)carbonyl group;
- R⁴ and R⁷ represent, independently of one another, a hydrogen atom, a hydroxyl group, a C₁-C₄ alkyl group, an amino group, a C₁-C₄ alkoxy group, a (C₂-C₄ acyl)oxy group or a (C₂-C₄ acyl)amino group;
- R⁵ represents a hydrogen atom, a hydroxyl group, a C₁-C₄ alkoxy group, a C₁-C₄ alkyl group, a halogen atom, an amino group, a (C₂-C₁₄ acyl)oxy group, a (C₂-C₄ acyl)amino group or a trimethylsilyloxy group;
- R⁶ represents a hydrogen atom, a hydroxyl group, a C₁-C₄ alkoxy group, an amino group, a (C₂-C₄ acyl)oxy group, a (C₂-C₄ acyl)amino group, a trimethylsilyloxy group or a hydroxy(C₂-C₄ alkyl)amino group;
- it not being possible for R⁵ and R⁶ simultaneously to designate a C₁-C₄ alkoxy radical;
- it also being possible for R⁵ and R⁶, together with the carbon atoms to which they are attached, to form a carbonyldioxy ring;
- at least one of the radicals R⁴ to R⁷ represents a group OZ or NHR⁸, at most one of the radicals R⁴ to R⁷ representing NHR⁸ and at most two of the radicals R⁴ to R⁷ representing OZ, and, in the case where Z represents a hydrogen atom, the two OH groups are in positions 5 and 6; and at least one of the radicals R⁴ to R⁷ represents a hydrogen atom and, in the case where only one of these radicals R⁴ to R⁷ represents a hydrogen atom, then only one radical from among R⁴ to R⁷ then represents NHR⁸ or OZ, the other radicals representing a C₁-C₄ alkyl group;
- the radical R⁸ of the group NHR⁸ denoting a hydrogen atom or a C₂-C₄ acyl or C₂-C₄ hydroxyalkyl group, and the radical Z of the group OZ denoting a hydrogen atom, a C₂-C₁₄ acyl group, a C₁-C₄ alkyl group or a trimethylsilyl group,
and their alkali metal, alkaline earth metal, ammonium or amine salts.

2. Use according to Claim 1, characterized in that the indole compound of formula (I) is chosen from: 4-hydroxyindole, 5-hydroxyindole, 6-hydroxyindole, 7-hydroxyindole, 4-hydroxy-5-methoxyindole, 4-hydroxy-5-ethoxyindole, 2-carboxy-5-hydroxyindole, 5-hydroxy-6-methoxyindole, 6-hydroxy-7-methoxyindole, 5-methoxy-6-hydroxyindole, 5,6-dihydroxyindole, N-methyl-5,6-dihydroxyindole, 2-methyl-5,6-dihydroxyindole, 3-methyl-5,6-dihydroxyindole, 2,3-dimethyl-5,6-dihydroxyindole, 2-carboxy-5,6-dihydroxyindole, 4-hydroxy-5-methylindole, 2-carboxy-6-hydroxyindole, 6-hydroxy-N-methylindole, 2-ethoxycarbonyl-5,6-dihydroxyindole, 4-hydroxy-7-methoxy-2,3-dimethylindole, 4-hydroxy-5-ethoxy-N-methylindole, 6-hydroxy-5-methoxy-2-methylindole, 6-hydroxy-5-methoxy-2,3-dimethylindole, 6-hydroxy-2-ethoxycarbonylindole, 7-hydroxy-3-methylindole, 5-hydroxy-6-methoxy-2,3-dimethylindole, 5-hydroxy-3-methylindole, 5-acetoxy-6-hydroxyindole, 5-hydroxy-2-ethoxycarbonylindole, 6-hydroxy-2-carboxy-5-methylindole, 6-hydroxy-2-ethoxycarbonyl-5-methoxyindole, 6-[N-(β-hydroxyethyl)amino]-indole, 4-aminoindole, 5-aminoindole, 6-aminoindole, 7-aminoindole, N-methyl-6-(β-hydroxy-ethylamino)indole, 6-amino-2,3-dimethylindole, 6-amino-2,3,4,5-tetramethylindole, 6-amino-2,3,4-trimethylindole, 6-amino-2,3,5-trimethylindole, 6-amino-2,3,6-trimethylindole, 5,6-diacetoxyindole, 5-methoxy-6-acetoxyindole, 5,6-trimethylsilyloxyindole, 5,6-dihydroxyindole phosphoric ester, 5,6-dibenzyloxyindole and the addition salts of these compounds.

3. Use according to Claim 1, characterized in that the indole compound is chosen from: 5,6-dihydroxyindole, 6-hydroxyindole, 7-hydroxyindole, 2-methyl-5,6-dihydroxy-indole hydrobromide, 7-aminoindole, 3-methyl-5,6-dihydroxyindole, 4-hydroxy-5-methoxyindole and 2,3-dimethyl-5-methoxy-6-hydroxyindole.

4. Use according to any one of Claims 1 to 3, characterized in that the oxidative polymerization to form the pigment is performed in an aqueous, water/solvent(s) or solvent(s) medium in the air, in the presence or absence of an alkaline agent and/or of an oxidizing agent.

5. Use according to Claim 4, characterized in that the oxidizing agent is chosen from hydrogen peroxide, hydrogen peroxide used in the presence of an alkaline agent, hydrogen peroxide used in the presence of an iodide ion, periodic acid, its water-soluble salts and its derivatives, permanganates, dichromates, sodium hypochloride, ammonium persulphate and sodium nitrite.

6. Use according to Claim 4, characterized in that the oxidizing agent is chosen from ortho- and parabenzoquinones, ortho- or para-benzoquinone mono- or diimines, 1,2- and 1,4-naphthoquinone and 1,2- and 1,4-naphthoquinone mono- or diimines.

7. Use according to any one of Claims 1 to 4, characterized in that the pigment results from oxidative polymerization performed in an aqueous and/or water/solvent(s) medium with hydrogen peroxide in the presence of aqueous ammonia.

8. Use according to any one of Claims 1 to 7, characterized in that the particle size of the pigment particles is such that 90% of particles have a diameter of less than 100 µm, and preferably less than 50 µm.

9. Use according to any one of Claims 1 to 8, characterized in that the pigment resulting from the oxidative polymerization of the indole compounds of formula (I) has a particle size such that the average diameter of the particles is less than 50 µm, and preferably less than 20 µm.

10. Use according to any one of Claims 1 to 9, characterized in that the insoluble pigment resulting from the oxidative polymerization of indole derivatives of formula (I) is used in a composition, comprising a medium suitable for application of the pigment to the fibres, in a proportion of between 0.05 and 10%, and preferably 0.2 and 2%, by weight relative to the total weight of the composition.

11. Use according to Claim 10, characterized in that the medium suitable for temporary dyeing is an aqueous medium which can contain one or more solvents chosen from lower alcohols, polyols, glycol ethers, esters or mixtures thereof.

12. Use according to Claims 10 and 11, characterized in that the composition contains a suspending or thickening agent enabling the insoluble pigments to be distributed homogeneously in the composition and on the treated fibres.

13. Use according to Claim 12, characterized in that the suspending or thickening agent is chosen from cellulose derivatives, biopolysaccharides, guar gum or inorganic thickeners.

14. Use according to one of Claims 10 to 13, characterized in that the medium suitable for temporary dyeing contains a film-forming latex.

15. Composition intended for use for the temporary dyeing of keratinous fibres, characterized in that it contains, in a medium suitable for temporary dyeing, at least one isolated, insoluble pigment resulting from the oxidative polymerization of indole derivatives corresponding to the formula (I) 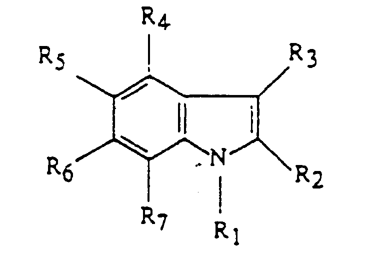 in which:
- R¹ and R³ represent, independently of one another, a hydrogen atom or a C₁-C₄ alkyl group;
- R² represents a hydrogen atom, a C₁-C₄ alkyl group, a carboxyl group or a (C₁-C₄ alkoxy)carbonyl group;
- R⁴ and R⁷ represent, independently of one another, a hydrogen atom, a hydroxyl group, a C₁-C₄ alkyl group, an amino group, a C₁-C₄ alkoxy group, a (C₂-C₄ acyl)oxy group or a (C₂-C₄ acyl)amino group;
- R⁵ represents a hydrogen atom, a hydroxyl group, a C₁-C₄ alkoxy group, a C₁-C₄ alkyl group, a halogen atom, an amino group, a (C₂-C₁₄ acyl)oxy group, a (C₂-C₄ acyl)amino group or a trimethylsilyloxy group;
- R⁶ represents a hydrogen atom, a hydroxyl group, a C₁-C₄ alkoxy group, an amino group, a (C₂-C₄ acyl)oxy group, a (C₂-C₄ acyl)amino group, a trimethylsilyloxy group or a hydroxy(C₂-C₄ alkyl)amino group;
- it not being possible for R⁵ and R⁶ simultaneously to designate a C₁-C₄ alkoxy radical;
- it also being possible for R⁵ and R⁵, together with the carbon atoms to which they are attached, to form a carbonyldioxy ring;
- at least one of the radicals R⁴ to R⁷ represents a group OZ or NHR⁸, at most one of the radicals R⁴ to R⁷ representing NHR⁸ and at most two of the radicals R⁴ to R⁷ representing OZ, and, in the case where Z represents a hydrogen atom, the two OH groups are in positions 5 and 6; and at least one of the radicals R⁴ to R⁷ represents a hydrogen atom and, in the case where only one of these radicals R⁴ to R⁷ represents a hydrogen atom, then only one radical from among R⁴ to R⁷ then represents NHR⁸ or OZ, the other radicals representing a C₁-C₄ alkyl group;
- the radical R8 of the group NHR⁸ denoting a hydrogen atom or a C₂-C₄ acyl or C₂-C₄ hydroxyalkyl group, and the radical Z of the group OZ denoting a hydrogen atom, a C₂-C₁₄ acyl group, a C₁-C₄ alkyl group or a trimethylsilyl group,
and their alkali metal, alkaline earth metal, ammonium or amine salts
and at least one suspending or thickening agent chosen from carboxymethylcellulose, hydroxyethylcellulose, hydroxypropylcellulose; xanthan gums or sceroglucans, guar gum; inorganic thickeners; the products, crosslinked or otherwise, resulting from the ionic interaction of a cationic polymer, formed from a copolymer of cellulose or of a cellulose derivative grafted with a water-soluble quaternary ammonium monomer salt, and a carboxylic anionic polymer; aqueous dispersions of ammonium acrylate/acrylamide copolymers crosslinked with a crosslinking agent containing olefinic unsaturation, dispersed in a water-in-oil emulsion; copolymers of methyl vinyl ether and maleic anhydride, crosslinked or otherwise; crosslinked polyacrylic acids; crosslinked copolymers of acrylic acid and long-chain (C10-C30) alkyl methacrylate or acrylate.

16. Composition according to Claim 15, characterized in that the suspending or thickening agent is used in proportions of between 0.05 and 10% by weight relative to the total weight of the composition.

17. Composition intended for use for the temporary dyeing of keratinous fibres, characterized in that it contains, in a medium suitable for temporary dyeing, at least one isolated, insoluble pigment originating from the oxidative polymerization of the compound of formula (I) as defined in Claim 1, and a film-forming latex.

18. Composition according to Claim 17, characterized in that the film-forming latex is chosen from homopolymers and copolymers of vinyl acetate; homopolymers and copolymers derived from acrylic acid; polyurethanes; butadiene/styrene copolymers, carboxylated or otherwise; butadiene/acrylonitrile copolymers, carboxylated or otherwise; styrene/acrylic esters copolymers; vinyl acetate/acrylic esters copolymers; styrene/butadiene/ vinylpyridine terpolymers; copolymers of vinyl chloride or of vinylidene chloride.

19. Use according to any one of Claims 1 to 14, characterized in that the insoluble pigment resulting from the oxidative polymerization of the compounds of formula (I) is employed in the form of a composition as is defined in any one of Claims 15 to 18.

20. Use according to any one of Claims 1 to 14 and 19, characterized in that the temporary dyeing composition contains, in addition, volatile or nonvolatile, insoluble or soluble silicones in the form of oils, gums, resins or powders; nonionic, anionic, cationic or amphoteric polymers; proteins, quaternized or otherwise; sunscreen agents; surfactants; antifoams; hydrating agents; humectants; emollients; vegetable or synthetic oils; preservatives; sequestering agents; antioxidants; pearlescence agents; perfumes; alkalinizing or acidifying agents; pigments; direct dyes.

21. Process for the temporary dyeing of keratinous fibres, characterized in that there is applied to these fibres a composition containing, in a medium suitable for the dyeing of these fibres, an isolated, insoluble pigment resulting from the oxidative polymerization of the indole compounds as are defined in any one of Claims 1 to 3.

## Patentansprüche

1. Verwendung eines unlöslichen isolierten Pigments zur zeitlichen Färbung keratinischer Fasern, das erhalten wird nach oxidativer Polymerisation einer Indolverbindung der Formel (I): 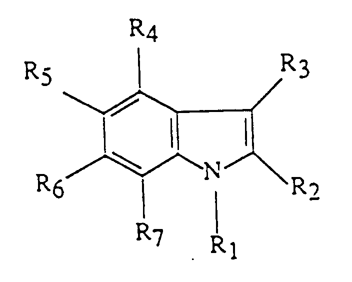 worin gilt:
- R¹ und R³ stellen, unabhängig voneinander, ein Wasserstoffatom oder eine C₁₋₄-Alkylgruppe dar;
- R² stellt ein Wasserstoffatom, eine C₁₋₄-Alkyl-, Carboxyl- oder eine C₁₋₄-Alkoxycarbonylgruppe dar;
- R⁴ und R⁷ stellen, unabhängig voneinander, ein Wasserstoffatom, eine Hydroxy-, C₁₋₄-Alkyl-, Amino-, C₁₋₄-Alkoxy-, C₂₋₄-Acyloxy- oder eine C₂₋₄-Acylaminogruppe dar;
- R⁵ stellt ein Wasserstoffatom, eine Hydroxy-, C₁₋₄-Alkoxy-, C₁₋₄-Alkylgruppe, ein Halogenatom, eine Amino-, C₂₋₁₄-Acyloxy-, C₂₋₄-Acylamino- oder eine Trimethylsilyloxygruppe dar;
- R⁶ stellt ein Wasserstoffatom, eine Hydroxy-, C₁₋₄-Alkoxy-, Amino-, C₂₋₄-Acyloxy-, C₂₋₄-Acylamino-, Trimethylsilyloxy- oder eine Hydroxy-C₂₋₄-alkylaminogruppe dar;
- R⁵ und R⁶ bedeuten nicht gleichzeitig einen C₁₋₄-Alkoxyrest;
- R⁵ und R⁶ können, zusammen mit den Kohlenstoffatomen, an die sie gebunden sind, auch einen Carbonyldioxy-Ring bilden;
- mindestens einer der Reste R⁴ bis R⁷ stellt eine OZ- oder NHR⁸-Gruppierung dar, wobei höchstens einer der Reste R⁴ bis R⁷ NHR⁸ und höchstens zwei der Reste R⁴ bis R⁷ OZ darstellen, und, wenn Z ein Wasserstoffatom darstellt, die beiden OH-Gruppen in den Positionen 5 und 6 vorliegen; und mindestens einer der Reste R⁴ bis R⁷ stellt ein Wasserstoffatom dar, und wenn ein einziger dieser Reste R⁴ bis R⁷ ein Wasserstoffatom darstellt, dann stellt ein einziger Rest unter R⁴ bis R⁷ NHR⁸ oder OZ dar, wobei die anderen Reste eine C₁₋₄-Alkylgruppe darstellen;
- der Rest R⁸ der NHR⁸-Gruppierung bedeutet ein Wasserstoffatom, eine C₂₋₄-Acyl- oder C₂₋₄-Hydroxyalkylgruppe, und der Rest Z der OZ-Gruppierung bedeutet ein Wasserstoffatom, eine C₂₋₄-Acyl-, C₁₋₄-Alkyl-oder eine Trimethylsilylgruppe,
wobei deren Alkali-, Erdalkali-, Ammonium- oder Aminsalze eingeschlossen sind.

2. Verwendung gemäß Anspruch 1,
dadurch **gekennzeichnet,** daß
die Indolverbindung der Formel (I) ausgewählt ist aus: 4-Hydroxyindol, 5-Hydroxyindol, 6-Hydroxyindol, 7-Hydroxyindol, 4-Hydroxy-5-methoxyindol, 4-Hydroxy-5-ethoxyindol, 2-Carboxy-5-hydroxyindol, 5-Hydroxy-6-methoxyindol, 6-Hydroxy-7-methoxyindol, 5-Methoxy-6-hydroxyindol, 5,6-Dihydroxyindol, N-Methyl-5,6-dihydroxyindol, 2-Methyl-5,6-dihydroxyindol, 3-MEthyl-5,6-dihydroxyindol, 2,3-Dimethyl-5,6-dihydroxyindol, 2-Carboxy-5,6-dihydroxyindol, 4-Hydroxy-5-methylindol, 2-Carboxy-6-hydroxyindol, 6-Hydroxy-N-methylindol, 2-Ethoxycarbonyl-5,6-dihydroxyindol, 4-Hydroxy-7-methoxy-2,3-dimethylindol, 4-Hydroxy-5-ethoxy-N-methylindol, 6-Hydroxy-5-methoxy-2-methylindol, 6-Hydroxy-5-methoxy-2,3-dimethylindol, 6-Hydroxy-2-ethoxycarbonylindol, 7-Hydroxy-3-methylindol, 5-Hydroxy-6-methoxy-2,3-dimethylindol, 5-Hydroxy-3-methylindol, 5-Acetoxy-6-hydroxyindol, 5-Hydroxy-2-ethoxycarbonylindol, 6-Hydroxy-2-carboxy-5-methylindol, 6-Hydroxy-2-ethoxycarbonyl-5-methoxyindol, 6-N,β-Hdroxyethylaminoindol, 4-Aminoindol, 5-Aminoindol, 6-Aminoindol, 7-Aminoindol, N-Methyl-6,β-hydroxyethylainoindol, 6-Amino-2,3-dimethylindol, 6-Amino-2,3,4,5-tetramethylindol, 6-Amino-2,3,4-trimethylindol, 6-Amino-2,3,5-trimethylindol, 6-Amino-2,3,6-trimethylindol, 5,6-Diacetoxyindol, 5-Mehoxy-6-acetoxyindol, 5,6-Trimethylsilyloxyindol, Phosphorester von 5,6-Dihyroxyindol, 5,6-Dibenzyloxyindol sowie aus den Additionssalzen dieser Verbindung.

3. Verwendung gemäß Anspruch 1,
dadurch **gekennzeichnet,** daß
die Indolverbindung ausgewählt ist aus: 5,6-Dihydroxyindol, 6-Hydroxyindol, 7-Hydroxyindol, 2-Methyl-5,6-dihydroxyindol-Hdrobromid, 7-Aminoindol, 3-Methyl-5,6-dihydroxyindol, 4-Hydroxy-5-methoxyindol, 2,3-Dimethyl-5-methoxy-6-hydroxyindol.

4. Verwendung gemäß jedem der Ansprüche 1 bis 3,
dadurch **gekennzeichnet,** daß
die oxidierende Polymerisation zur Bildung des Pigments in einem Milieu aus Wasser, Wasser/Lösungsmittel(n) oder aus Lösungsmittel(n) an Luft in Gegenwart oder Abwesenheit eines alkalischen Mittels und/oder eines oxidierenden Mittels durchgeführt wird.

5. Verwendung gemäß Anspruch 4,
dadurch **gekennzeichnet,** daß
das oxidierende Mittel aus Wasserstoffperoxid, in Gegenwart eines alkalischen Mittels verwendetem Wasserstoffperoxid, in Gegenwart eines Jodidions verwendetem Wasserstoffperoxid, aus Perjodsäure und seinen wasserlöslichen Salzen und Derivaten, Permanganaten, Bichromaten, Natriumhypochlorit Ammoniumpersulfat und aus Natriumnitrit ausgewählt ist.

6. Vewendung gemäß Anspruch 4,
dadurch **gekennzeichnet,** daß
das oxidierende Mittel aus o- und p-Benzochinonen, o- oder p-Benzochinonmono- oder -diiminen, 1,2- und 1,4-Naphthochinon, 1,2- und 1,4-Naphthochinonmono- oder -diiminen ausgewählt ist.

7. Verwendung gemäß einem der Ansprüche 1 bis 4,
dadurch **gekennzeichnet,** daß
das Pigment aus einer oxidierenden Polymerisation resultiert, die in einem Milieu aus Wasser und/oder Wasser/Lösungsmittel(n) mit Wasserstoffperoxid in Gegenwart von Ammoniak durchgeführt worden ist.

8. Verwendung gemäß jedem der Ansprüche 1 bis 7,
dadurch **gekennzeichnet,** daß
die Korngröße der Pigmentpartikel so beschaffen ist, daß 90% der Partikel einen Durchmesser von weniger als 100 und vorzugsweise von weniger als 50 µm aufweisen.

9. Verwendung gemäß jedem der Ansprüche 1 bis 8,
dadurch **gekennzeichnet,** daß
das aus der oxidierenden Polymerisation der Indolverbindungen der Formel (I) entstehende Pigment eine Korngröße eines mittleren Durchmessers der Teilchen von weniger als 50 und vorzugsweise von weniger als 20 µm aufweist.

10. Vewendung gemäß jedem der Ansprüche 1 bis 9,
dadurch **gekennzeichnet,** daß
das aus der oxidierenden Polymerisation der Indolderivate der Formel (I) entstehende unlöslche Pigment in einer Zusammensetzung, enthaltend ein zur Aufbringung des Pigments auf Fasern geeignetes Milieu, in einem Mengenanteil von 0,5 bis 10 und vorzugsweise von 0,2 bis 2 Gew.% verwendet wird, bezogen auf das Gesamtgewicht der Zusammensetzung.

11. Verwendung gemäß Anspruch 10,
dadurch **gekennzeichnet,** daß
das zur zeitlichen Färbung geeignete Milieu ein wässriges Milieu ist, das ein oder mehrere Lösungsmittel enthalten kann, die aus Niedrigalkoholen, Polyolen, Ethern von Glycolen, Estern oder deren Mischungen ausgewählt sind.

12. Verwendung gemäß der Ansprüche 10 oder 11,
dadurch **gekennzeichnet,** daß
die Zusammensetzung ein Mittel zur Suspendierung oder ein Verdickungsmittel enthält, welche es ermöglichen, die unlöslichen Pigmente in der Zusammensetzung und auf den behandelten Fasern in homogener Weise zu verteilen.

13. Verwendung gemäß Anspruch 12,
dadurch **gekennzeichnet,** daß
das Suspendier- oder Verdickungsmittel aus Cellulosederivaten, Biopolysacchariden, Guar-Gummi oder aus mineralischen Verdickungsmitteln ausgewählt sind.

14. Verwendung gemäß einem der Ansprüche 10 bis 13,
dadurch **gekennzeichnet,** daß
das zur zeitlichen Färbung geeignete Milieu einen filmbildenden Latex enthält.

15. Zusammensetzung zur Verwendung zur zeitlichen Färbung keratinischer Fasern,
dadurch **gekennzeichnet,** daß
sie in einem zur zeitlichen Färbung geeigneten Milieu mindestens ein unlösliches isoliertes Pigment, das sich aus der oxidierenden Polymerisation von Indolderivaten der Formel (I) ergibt, 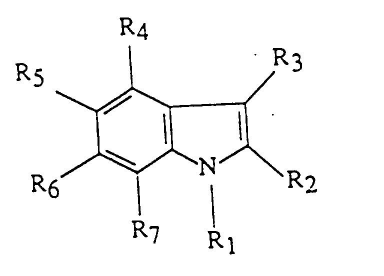 worin gilt:
- R¹ und R³ stellen, unabhängig voneinander, ein Wasserstoffatom oder eine C₁₋₄-Alkylgruppe dar;
- R² stellt ein Wasserstoffatom, eine C₁₋₄-Alkyl-, Carboxyl- oder eine C₁₋₄-Alkoxycarbonylgruppe dar;
- R⁴ und R⁷ stellen, unabhängig voneinander, ein Wasserstoffatom, eine Hydroxy-, C₁₋₄-Alkyl-, Amino-, C₁₋₄-Alkoxy-, C₂₋₄-Acyloxy- oder eine C₂₋₄-Acylaminogruppe dar;
- R⁵ stellt ein Wasserstoffatom, eine Hydroxy-, C₁₋₄-Alkoxy-, C₁₋₄-Alkylgruppe, ein Halogenatom, eine Amino-, C₂₋₁₄-Acyloxy-, C₂₋₄-Acylamino- oder eine Trimethylsilyloxygruppe dar;
- R⁶ stellt ein Wasserstoffatom, eine Hydroxy-, C₁₋₄-Alkoxy-, Amino-, C₂₋₄-Acyloxy-, C₂₋₄-Acylamino-, Trimethylsilyloxy- oder eine Hydroxy-C₂₋₄-alkylaminogruppe dar;
- R⁵ und R⁶ bedeuten nicht gleichzeitig einen C₁₋₄-Alkoxyrest;
- R⁵ und R⁶ können, zusammen mit den Kohlenstoffatomen, an die sie gebunden sind, auch einen Carbonyldioxy-Ring bilden;
- mindestens einer der Reste R⁴ bis R⁷ stellt eine OZ- oder NHR⁸-Gruppierung dar, wobei höchstens einer der Reste R⁴ bis R⁷ NHR⁸ und höchstens zwei der Reste R⁴ bis R⁷ OZ darstellen, und, wenn Z ein Wasserstoffatom darstellt, die beiden OH-Gruppen in den Positionen 5 und 6 vorliegen; und mindestens einer der Reste R⁴ bis R⁷ stellt ein Wasserstoffatom dar, und wenn ein einziger dieser Reste R⁴ bis R⁷ ein Wasserstoffatom darstellt, dann stellt ein einziger Rest unter R⁴ bis R⁷ NHR⁸ oder OZ dar, wobei die anderen Reste eine C₁₋₄-Alkylgruppe darstellen;
- der Rest R⁸, der NHR⁸-Gruppierung bedeutet ein Wasserstoffatom, eine C₂₋₄-Acyl- oder C₂₋₄-Hydroxyalkylgruppe, und der Rest Z der OZ-Gruppierung bedeutet ein Wasserstoffatom, eine C₂₋₁₄-Acyl-, C₁₋₄-Alkyl-oder eine Trimethylsilylgruppe,
wobei deren Alkali-, Erdalkali-, Ammonium- oder Aminsalze eingeschlossen sind,
sowie mindestens ein Suspendier- oder Verdickungsmittel enthält, ausgewählt aus Carboxymethylcellulose, Hydroxyethylcellulose, Hydroxypropylcellulose, Gummiprodukten von Xanthan oder Scleroglucanen, Guar-Gummi, mineralischen Verdickungsmitteln, gegebenenfalls vernetzten Produkten, die sich aus der ionischen Wechselwirkung eines kationischen Polymer aus einem Copolymer von Cellulose oder einem Cellulosederivat, welche mit einem wasserlöslichen Monomersalz einer quaternären Ammoniumverbindung gepfropft sind, mit einem anionischen Carboxyl-Polymer ergeben, aus wässrigen Dispersionen von Copolymeren aus Ammoniumacrylat/Acrylamid, welche mit einem Vernetzungsmittel mit olefinischer Mehrfachungesättigtheit vernetzt sind, dispergiert in einer Wasser-in-Öl-Emulsion, aus gegebenenfalls vernetzten Copolymeren von Methylvinylether und Maleinanhydrid, aus vernetzten Polyacrylsäuren sowie aus vernetzten Copolymeren von Acrylsäure und C₁₀₋₃₀-Alkyl(meth)acrylat.

16. Zusammensetzung gemäß Anspruch 15,
dadurch **gekennzeichnet,** daß
das Suspendier- oder Verdickungsmittel in Mengenanteilen von 0,05 bis 10 Gew.% verwendet werden, bezogen auf das Gesamtgewicht der Zusammensetzung.

17. Zusammensetzung zur Verwendung zur zeitlichen Färbung keratinischer Fasern,
dadurch **gekennzeichnet,** daß
sie in einem zur zeitlichen Färbung geeigneten Milieu mindestens ein unlösliches isoliertes Pigment aus der oxidierenden Polymerisation der in Anspruch 1 definierten Verbindung der Formel (I) und einen filmbildenden Latex enthält.

18. Zusammensetzung gemäß Anspruch 17,
dadurch **gekennzeichnet,** daß
der filmbildende Latex aus Homopolymeren und Polymeren von Vinylacetat, von Acrylsäure abgeleiteten Homopolymeren und Copolymeren, Polyurethanen, Butadien/Styrol-Copolymeren, carboxyliert oder nicht, Butadien/Acrylnitril-Copolymeren, carboxyliert oder nicht, Styrol/Acrylester-Copolymeren, Vinylacetat/Acrylester-Copolymeren, Styrol/Butadien/Vinylpyridin-Terpolymeren sowie aus Copolymeren von Vinyl- oder Vinylidenchlorid ausgewählt ist.

19. Verwendung gemäß jedem der Ansprüche 1 bis 14,
dadurch **gekennzeichnet,** daß
das unlösliche Pigment aus der oxidierenden Polymerisation von Verbindungen der Formel (I) in Form einer in jedem der Ansprüche 15 bis 18 definierten Zusammensetzung angewandt wird.

20. Verwendung gemäß jedem der Ansprüche 1 bis 14 und 19,
dadurch **gekennzeichnet,** daß
die Zusammensetzung zur zeitlichen Färbung ausserdem unlösliche oder lösliche, flüchtige oder nicht-flüchtige Silikone, in Form von Ölen, Gummiprodukten, Harzen oder Pulvern, nicht-ionische, anionische, kationische oder amphotere Polymere, Proteine, quaterniert oder nicht, Sonnenfilterstoffe, oberflächenaktive Mittel, Antischaummittel, Hydratisiermittel, Feuchtigkeitsmittel, weichmachende Mittel, pflanzliche oder synthetische Öle, Konservierungsstoffe, Sequestriermittel, Antioxidantien, Perlmuttglanzmittel, Parfüm-Produkte, alkalisch oder sauer stellende Mittel, Pigmente und Direktfarbstoffe enthält.

21. Verfahren zur zeitlichen Färbung keratinischer Fasern,
dadurch **gekennzeichnet,** daß
man auf diese Fasern eine Zusammensetzung aufbringt, die, in einem zur Färbung dieser Fasern geeigneten Milieu, ein unlösliches isoliertes Pigment enthält, das sich aus der oxidierenden Polymerisation der in jedem der Ansprüche 1 bis 3 definierten Indolverbindungen ergibt.
